# EUROPEAN PATENT SPECIFICATION

(11) **EP 0 414 350 B1**
(45) Date of publication and mention of the grant of the patent: **24.08.1994**
(21) Application number: 90306747.8
(22) Date of filing: 20.06.1990
(51) Int. Cl.: A61M 25/10

(54) **Pleated balloon dilatation catheter and method of manufacture**
Plissierter Ballondilatationskatheter und Verfahren zur Herstellung
Cathéter dilatateur à ballonnet plissé et procédé de fabrication

(30) Priority: 25.08.1989 US 398630
(43) Date of publication of application: 27.02.1991
(73) Proprietor: C.R. BARD, INC., Murray Hill New Jersey 07974 (US)
(72) Inventor: Davey, Christopher T., Boston Massachusetts 02130 (US)
(74) Representative: Woodward, John Calvin

(56) References cited:
- EP-A- 0 376 451
- US-A- 30 365
- US-A- 4 141 364

## Description

### FIELD OF THE INVENTION

This invention relates to balloon catheters used for dilatation procedures.

### BACKGROUND OF THE INVENTION

Balloon dilatation catheters are used for a variety of medical procedures, including dilation of obstructed body lumens, such as blood vessels, coronary arteries and the esophageal tract. In particular, such catheters are used in angioplasty procedures to enlarge the lumen of a blood vessel which is constricted or stenosed by arteriosclerosis.

In a type of angioplasty known as percutaneous transluminal coronary angioplasty (PTCA), dilatation catheters are used in conjunction with a guide catheter through which the dilatation catheter is inserted and guided to the desired location in the body lumen. In a typical PTCA procedure, the guide catheter is percutaneously introduced into the patient's arterial system and is fluoroscopically guided to the entrance to the coronary artery. A dilatation catheter, having a balloon at its distal end, then is passed through the guide catheter, so that the balloon extends beyond the distal end of the guide catheter into the coronary artery. The catheter then is manipulated to place the balloon (deflated) into the obstruction. Once the balloon is placed within the obstruction, it is inflated to dilate the obstructed lumen.

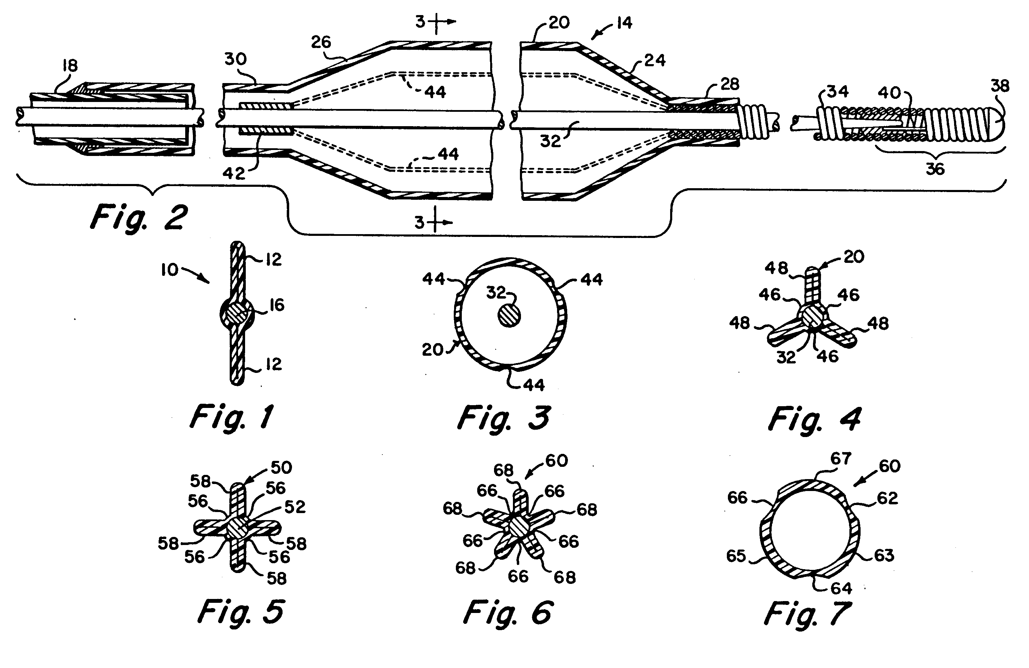

The balloon of the dilatation catheter must be deflated to a low profile in order for it to be passed through the guide catheter and, more particularly, through the stenosis. The balloon is deflated by applying negative pressure to the balloon through an inflation/deflation lumen that extends from the proximal end of the catheter to the interior of the balloon. The configuration assumed by the balloon upon deflation presents a problem in catheter utilization. Typically, the balloon forms a pair of opposed, radially-extending, flat wings, when collapsed under the influence of negative pressure, as shown in Fig. 1. To insert the balloon into the lumen of a guide catheter, the physician must manually wrap the wings about the catheter shaft prior to insertion into the guide catheter. The reduced profile of the balloon, caused by the wrapping, facilitates insertion of the balloon into the guide catheter and the obstruction. However, after the balloon has been inflated in the patient and it is desired to recollapse the balloon, the balloon will again tend to assume the two wing configuration. The radial extent of the opposed wings may make it difficult for the balloon to refold as it is withdrawn into the guide catheter or if it is desired to recollapse the balloon and advance it to another stenosed location. In the latter case, the radial extending wings may make it more difficult for the balloon to be inserted into the stenosis.

A desirable feature of a balloon dilatation catheter, therefore, is a balloon which has a reduced profile to facilitate insertion into a guide catheter and a vascular stenosis, without the need to manually wrap the balloon about the catheter shaft.

The foregoing problem has not gone unrecognized as evidenced by proposed devices to provide a means for causing a dilatation balloon to be wrapped closely about a catheter shaft to form a reduced profile. For example, see U.S.-A-4,292,974, issued to Fogerty. Notwithstanding such proposals, there remains a need for a simple effective means to facilitate collapse of a dilatation balloon to a low profile.

A further problem encountered in using dilatation balloon catheters is the failure of the balloon to deflate, following dilatation of the obstruction. Because of a malfunction in the catheter itself, the dilatation balloon may fail to deflate when aspirated. In these instances, the balloon must be intentionally destroyed before withdrawal through the guide catheter. The balloon is destroyed by inflating the balloon to a pressure at which the balloon wall ruptures or bursts. Unfortunately, many current dilatation balloons have burst pressures which exceed the delivery capabilities of most clinical inflation devices, often requiring burst pressures in excess of 20 Bars. Such pressure may rupture the body lumen as well as the dilatation balloon, thereby creating an undesirable hazard to the patient. A further desirable feature of a dilatation catheter, therefore, is to provide a balloon having an artificially lower burst pressure to facilitate easy destruction of the balloon, if necessary.

EP-A-0376451 discloses a balloon for a dilatation catheter comprising a tubular polymeric member formed to collapse under the influence of reduced internal pressure into a configuration defined by at least three longitudinally extending wings.

The present invention according to claim 1 overcomes the problems of the prior art by providing a tubular body which has a segment of reduced thickness between adjacent wings to provide a folding pleat whereby the tubular body defines at least three longitudinally extending pleats and alternating wings.

The segments form fold lines, extending longitudinally of the balloon and causing the balloon to collapse into a pleated configuration when aspirated. When inflated, the pleats expand so that the balloon forms the cylindrical configuration desired for dilatation. The preferred pleated balloon of the present invention defines wings having shorter radial dimensions so the wings are mor easily collapsed. They define a reduced profile when collapsed to facilitate insertion and withdrawal through a guide catheter as well as a stenosis.

According to claim 7, a dilatation catheter including a balloon in accordance with the invention is further provided.

The invention according to claim 12 relates to a method of forming a pleated balloon comprising the steps of extruding a tube having at least three longitudinally extending segments of less wall thickness than the remainder of the tube to provide pleats therein and biaxially stretching the tube to a predefined shape.

A tubular extrusion of a polymeric material is formed having a plurality of circumferentially-spaced, longitudinally-extending lines defined by less wall thickness than the remainder of the tube. The balloon is formed by biaxially stretching the tubular extrusion, in accordance with the method described in US-A-4,440,421 issued to Levy.

The balloon of the invention incorporates improved means for collapsing the balloon to a low profile that defines at least three pleats and alternating wings.

The balloon of the preferred dilatation catheter, when withdrawn through the lumen of a guide catheter, reduces the risk of damage to the balloon wall.

Preferably, the balloon has a predetermined burst pressure so that if the balloon bursts, it does so along the length of the balloon.

Preferred embodiments of the invention will now be described, by way of example only, with reference to the accompanying drawings wherein:
Figure 1 is a cross-sectional illustration through a dilatation balloon showing the manner in which typical prior art dilatation balloons collapse to form a pair of diametrically opposed wings;
Figure 2 is an enlarged longitudinal sectional illustration of the distal end of a dilatation catheter of the present invention;
Figure 3 is a sectional illustration of the dilatation catheter balloon of Figure 1 as seen along lines 3-3 of Figure 2;
Figure 4 is a sectional illustration of the balloon of Figure 3 in which three pleats are formed when in an evacuated, collapsed configuration;
Figure 5 is a sectional illustration of an alternate embodiment of the balloon of Figure 3 in which four pleats are formed when in an evacuated, collapsed configuration;
Figure 6 is a sectional illustration of an alternate embodiment of the balloon of Figure 3 in which five pleats are formed when in an evacuated, collapsed configuration; and
Fig. 7 is a cross-sectional illustration of a tubular extrusion used in the method of manufacturing the pleated balloon of the present invention.

### DETAILED DESCRIPTION OF THE PREFERRED EMBODIMENT

Fig. 2 illustrates, diagrammatically, the cross-sectional configuration assumed by a typical dilatation balloon 10 when the balloon is aspirated by applying negative pressure to the balloon interior, causing it to deflate. Balloon 10 forms a pair of diametrically opposed wings 12 as the balloon collapses. The physician typically must manually wrap the wings 12 about the catheter shaft 16, creating a low profile configuration which permits easy insertion of the dilatation catheter into the lumen of a guide catheter. Difficulty may arise, following the dilation procedure, either when it is desired to withdraw the dilatation catheter through the lumen of the guide catheter or when it is desired to deflate the balloon and reposition it at another vascular location to perform another dilatation. In particular, the diametrically opposed wings 12 may not wrap closely about the catheter shaft as the catheter is withdrawn back into the guide catheter or is repositioned within the arteries. Instead, the wings may catch on the distal opening of the guide catheter or may preclude reinsertion of the balloon into another stenosis.

In accordance with the present invention, a deflated dilatation balloon has a low profile configuration having at least three pleats and alternating wings. When such a balloon is collapsed, each of the wings is relatively short, in its radial extent, and collapses more readily about the catheter shaft. When inflated, the balloon assumes a cylindrical configuration.

In accordance with one embodiment of the present invention,as shown in Figs 1 and 3, a dilatation catheter 14 includes an elongate flexible shaft 18 having a dilatation balloon 20 carried at the distal end of the shaft, An inflation lumen 22 extends through shaft 18 from the proximal to the distal end and is in fluid communication with the interior of balloon 20. The proximal end of the shaft (not shown) is adapted to be connected to a suitable fitting, such as a luer fitting, by which an inflation/deflation device such as a syringe may be connected. Shaft 18 may be formed from an appropriate polymeric material as will be appreciated by those familiar with the art.

Dilatation balloon 20 includes an elongate cylindrical portion having a pair of tapered conical sections 24 and 26 at its distal and proximal ends, respectively. A distal collar 28 and a proximal collar or an elongate sleeve 30 extend from conical ends 24 and 26, respectively. The dilatation balloon may be formed from a polymeric material such as polyethylene terephthalate and may be formed in accordance with the method of the present invention, as described hereinafter.

Proximal sleeve 30 is adhesively attached to the distal end of shaft 18 as shown in Fig. 2. A support wire 32 extends through lumen 22, the interior of balloon 20, and distally therebeyond. The annual space surrounding support wire 32 provides an annular inflation/deflation lumen in fluid communication with the interior of balloon 20. The support wire 32 has a tapered distal tip which extends into and is attached to a helically-wound radiopaque coil 34. Coil 34 has a distal tip 36 terminating with a rounded weld bead 38. The distal collar 28 of balloon 20 is adhesively attached to the proximal end of coil 34, as shown in Fig. 2. The distal tip 36 of coil 34 may include a bendable, stainless steel shaping ribbon 40, which is secured to the distal tip of support wire 32 at one end and to weld bead 38 at the other end.

Catheter 14 may include a radiopaque marker band 42, securely attached to support wire 34, near the proximal portion of balloon 20. The marker band provides a means by which the physician can fluoroscopically verify the position of balloon 20.

Fig. 3 is a sectional illustration of the dilatation balloon 20 as seen along lines 3-3 of Fig. 1. By way of example, in a catheter adapted for coronary arterial use, the balloon of the dilatation catheter illustrated may have an inflated diameter of between 1.5 mm to 4.0 mm. The cylindrical midportion of balloon 20 may be 2 cm long. The end cones 24 and 28 may be about 4 to about 7 mm long. It should be understood, however, that the invention contemplates use in dilatation balloons other than for coronary angioplasty use and that the dimensions of such other balloons may vary from those illustrated and described herein.

The wall of balloon 20 is relatively thin. By way of example, for a balloon having an inflated diameter of 3.0 mm, an appropriate wall thickness would be between about 0,0064mm (0,00025") to about 0,0127mm (0,00050"). The cones 24, 26 and collars 28, 30 may be thicker because they are expanded to a lesser degree than the cylindrical midportion of the balloon during the balloon fabrication process, discussed below.

As shown in Fig. 3, balloon 20 has a plurality of circumferentially spaced lines 44 of reduced wall thickness. Lines 44 extend longitudinally of balloon 20, as shown in in phantom in Fig. 2. For a dilatation balloon of about 3.0 mm diameter having a wall thickness from between 0,0064mm (0.00025") to 0,0127mm (0.00050"), the wall thickness at lines 44 may be approximately 0,0064mm (0.00025"). Lines 44 extend longitudinally of balloon 20 from distal collar 28 to proximal sleeve 30.

When balloon 20 is deflated by aspirating inflation lumen 22, the balloon collapses more easily along the lines 44 to create a substantially reduced profile characterized by three pleats 46 disposed intermediate three wings 48. It will be appreciated that the radial extension of each of the wings 48 of balloon 20 is considerably less than the radial extension of wings 12 in the unpleated balloon of Fig. 1.

The reduced profile assumed by balloon 20 upon evacuation not only minimizes the necessity for the balloon to be wrapped prior to insertion into the guide catheter, but substantially reduces the chance of damage to the balloon wall upon withdrawal through a guide catheter. It increases the ability of the deflated balloon to pass through tight stenoses. The reduced profile of balloon 20 prevents the balloon from being caught at the distal end of a guide catheter and substantially reduces the surface area of the balloon which is in contact with the guide catheter lumen during withdrawal.

Figs. 5 and 6 show alternate embodiments of the pleated dilatation balloon of Fig. 4. In Fig. 5, a pleated balloon 50 has a collapsed configuration about catheter shaft 52 which is characterized by four pleats 56 disposed intermediate wings 58. The folding of balloon 50 into the illustrated configuration results from four lines of reduced wall thickness formed along the balloon. Similarly, as shown in Fig. 6, a five pleat balloon 60 has a collapsed configuration about catheter shaft 62 which is characterized by five pleats 66 intermediate five wings 68. From a comparison of Figs. 4, 5 and 6, it can be appreciated that the profile of the pleated balloon decreases as the number of pleats increase. Generally, the larger the diameter of the balloon, the greater the number of pleats which may be formed into the balloon.

A further advantage of the present invention is that the lines extending longitudinally of the balloon may be manufactured to provide a minimum burst strength for the balloon. Presently, if a balloon does not deflate when desired, a practitioner may over inflate the balloon to intentionally rupture the balloon wall, causing immediate deflation of the balloon and enabling withdrawal of the balloon from the lumen. Lines 44 of Fig. 3 may be formed so as to serve as rupture points which would insure that the burst pressure of balloon 20 would not exceed the delivery capability of a selected clinical inflation device. A pleated balloon in accordance with the present invention may have a minimum burst pressure of 16 to 18 Bars compared to the 20 Bar minimum burst pressure of some dilatation balloons.

The invention is also useful in balloons where the nature of the material or balloon geometry is such that the balloon is susceptible to damage from handling and use. In some instances, it has been the practice to thicken the wall of such a balloon in order to make it more resistant to damage. An undesired by product of such a thickened balloon wall, however, may be that the burst pressure is increased far beyond that needed for the intended application. With the present invention, such a balloon may have the increased wall thickness while maintaining an acceptable burst pressure.

A method for making a pleated balloon in accordance with the present invention involves extruding a tube of polymeric material, such as polyvinyl chloride, polyethylene, or polyethylene terephthalate so as to have at least three circumferentially-spaced elongated segments of less wall thickness than the remainder of the tube. As shown in Fig. 7, a tube 60 is formed of relatively thick wall portions 63, 65 and 67 which are disposed intermediate relatively thin walled portions 62, 64 and 66. In the Fig. 7 embodiment, thin walled portions are defined by chordal flats formed in an angular spacing of 120° circumferentially about the tube. Each of the flats may define a chord corresponding to approximately 10% of the outer circumference of the tube. By way of example, for a polyethylene terephthalate tube intended to be formed into a 3.0 mm diameter balloon, the tube may have an inner diameter of about 0,429mm (0.0169"), having thick walled portions of about 0,0127mm (0.0050"), the thin walled portions of the tube should be about 0,096mm (0.0038"). The thin walled portions 62, 64 and 66 are implemented as thin lines which extend longitudinally of the extruded tube 60. A balloon formed from tube 60 preferentially collapses at thin walled portion 62, 64 and 66 while the thick walled portions 63, 65 and 67 project radially outward to form the wings.

Following extrusion of tube 60, the tube is then blow molded in accordance with the techniques disclosed in U.S.-A-4,490,421, and U.S. Patent Application Serial No. 001,759, filed January 9, 1987,the disclosures of which are incorporated herein by reference. Once tube 60 has been extruded, the tube is inserted into a mold chamber. Upon heating the chamber, the tube is heated by convection and radiation thereby softening the tube material. It will be appreciated that certain polymeric materials do not require heating to soften the tube material. Tube 60 is then axially stretched while positive pressure is simultaneously applied to the interior of the tube. The radial expansion, caused by the positive pressure on the tube interior, during axial stretching establishes biaxial stretching of the tube. The biaxial stretching is maintained until the tube assumes the shape of the mold.

It should be noted that the cones and neck portions of the balloon also are formed from the tube 60 having alternate thick and thin wall portions. As a result, the cones 24, 26 of the balloon also define a pleated arrangement and will tend to collapse into the pleated configuration when the balloon is aspirated. This may effectively reduce the angle made by the cone and may facilitate entry of the balloon into a tight stenosis. Additionally, by including the pleats in the cone regions, it may be possible to form balloons in which the cone regions fold down closely to the shaft.

From the foregoing, it will be appreciated that the invention provides a dilatation balloon having a tendency to collapse into a pleated configuration forming at least three or more pleats and alternating wings, the radial extension of such wings being relatively small. The pleated balloon folds more easily as it is withdrawn through the guide catheter.

Thus it will be appreciated that the invention provides a new and improved pleated balloon configuration for a dilatation catheter by which the balloon may be more readily contracted to a low profile, as well as a method for forming such a pleated balloon. Although the invention has been illustrated in connection with a coronary dilatation catheter, it may be used with other balloon catheters such as peripheral blood vessel dilatation catheters or esophageal catheter. Additionally, although the illustrative embodiment has been described in connection with a balloon made from polyethylene terephthalate, which is relatively inelastic, (non-compliant) the invention also may be incorporated in balloons formed from more compliant materials, such as polyvinyl chloride or polyethylene.

## Claims

1. A balloon for a dilatation catheter comprising a tubular polymeric member (20) formed to collapse under the influence of reduced internal pressure into a configuration defined by at least three longitudinally extending wings (68) characterised in that the tubular body (20) has a segment of reduced thickness between adjacent wings (68) to provide a folding pleat (66) whereby the tubular body (20) defines at least three longitudinally extending pleats (66) and alternating wings (68).

2. A balloon as claimed in claim 1 characterised in that the segments are lines extending longitudinally of the balloon (20).

3. A balloon as claimed in claim 1 or claim 2 characterised in that the balloon (20) is inelastic.

4. A balloon as claimed in claim 3 characterised in that the balloon (20) is formed from polyethylene terephthalate.

5. A balloon as claimed in claim 3 characterised in that the balloon (20) is formed from polyethylene.

6. A balloon as claimed in claim 3 characterised in that the balloon (20) is formed from polyvinyl chloride.

7. A dilatation catheter comprising an elongate flexible shaft (18) having at least one lumen (22) extending therethrough from its proximal to its distal end, a dilatation balloon (20) mounted on the distal end of the shaft (18) and in fluid communication with the lumen, and a fitting for connecting the lumen with an inflation and deflation device, the catheter being characterised in that the balloon (20) is as claimed in any of claims 1-6.

8. A dilatation catheter as claimed in claim 7 characterised in that the balloon, when deflated, forms said at least three pleats (66) about the shaft (18).

9. A dilatation catheter as claimed in claim 7 or claim 8 characterised in that the segments are fold lines extending longitudinally of the balloon (20).

10. A dilatation catheter as claimed in any of claims 7-9 characterised in that the catheter comprises an angioplasty catheter.

11. A dilatation catheter as claimed in claim 10 characterised in that the catheter comprises a percutaneous transluminal coronary angioplasty catheter.

12. A method for forming a pleated balloon for a dilatation catheter characterised by the steps of extruding a tube having at least three longitudinally extending segments of less thickness than the remainder of the tube to provide pleats (62,64,66) therein, and biaxially stretching the tube (20) to a predefined shape.

13. A method as claimed in claim 12 characterised in that the step of biaxially stretching the tube comprises axially stretching the tube while applying positive pressure to the interior of the tube at least during the axial stretching.

14. A method as claimed in claim 12 or claim 13 further characterised by the step of heating the tube at least after the step of extruding.

## Patentansprüche

1. Ballon für einen Dilatationskatheter, der ein rohrförmiges polymerisches Glied (20) umfasst, das so gebildet ist, dass es unter dem Einfluss von verringertem inneren Druck in eine Anordnung zusammensackt, die von wenigstens drei sich längs erstreckenden Flügeln (68) definiert ist, dadurch gekennzeichnet, dass der rohrförmige Körper (20) einen Abschnitt verringerter Dichte zwischen benachbarten Flügeln (68) hat, um eine sich faltende Falte (66) zu liefern, wobei der rohrförmige Körper wenigstens drei sich längs erstreckende Falten (66) und abwechselnde Flügel (68) definiert.

2. Ballon nach Anspruch 1, dadurch gekennzeichnet, dass die Abschnitte Linien sind, die sich längs des Ballons (20) erstrecken.

3. Ballon nach Anspruch 1 oder Anspruch 2, dadurch gekennzeichnet, dass der Ballon (20) inelastisch ist.

4. Ballon nach Anspruch 3, dadurch gekennzeichnet, dass der Ballon (20) aus Polyethylenterephthalat gebildet ist.

5. Ballon nach Anspruch 3, dadurch gekennzeichnet, dass der Balon (20) aus Polyethylen gebildet ist.

6. Ballon nach Anspruch 3, dadurch gekennzeichnet, dass der Ballon (20) aus Polyvinylchlorid gebildet ist.

7. Dilatationskatheter, das einen langgestreckten flexiblen Schaft (18) umfasst, der wenigstens Lumen (22) hat, das sich dadurch von seinem proximalen zu seinem distalen Ende erstreckt, einen auf dem distalen Ende des Schaftes (18) angebrachten Dilatationsballon (20) und der in Flüssigkeitsverbindung mit dem Lumen ist, und ein Verbindungsstück, um das Lumen an ein Aufblas- und Ablassgerät anzuschliessen, wobei der Katheter dadurch gekennzeichnet ist, dass der Ballon wie in einem der Ansprüche 1-6 ist.

8. Dilatationskatheter nach Anspruch 7, dadurch gekennzeichnet, dass der Ballon wenigstens drei Falten (66) um den Schaft (18) bildet, wenn er abgelassen ist.

9. Dilatationskatheter nach Anspruch 7 oder Anspruch 8, dadurch gekennzeichnet, dass die Abschnitte Falzlinien sind, die sich längs des Ballons (20) erstrecken.

10. Dilatationskatheter nach einem der Ansprüche 7-9, dadurch gekennzeichnet, dass der Katheter ein Gefässplastikkatheter umfasst.

11. Dilatationskatheter nach Anspruch 10, dadurch gekennzeichnet, dass der Katheter einen perkutanen durchlässigen Koronargefässplastikkatheter umfasst.

12. Verfahren zum Bilden eines plissierten Ballons für einen Dilatationskatheter, gekennzeichnet durch die Schritte, ein Rohr, das wenigstens drei sich längs erstreckende Abschnitte mit geringerer Dicke als der Rest des Rohres hat, strangzupressen, um Falten (62,64,66) darin zu liefern, und das Rohr (20) in eine vorbestimmte Gestalt biaxial zu strecken.

13. Verfahren nach Anspruch 12, dadurch gekennzeichnet, dass der Schritt, das Rohr biaxial zu strecken, umfasst, das Rohr axial zu strecken, während wenigstens während des axialen Streckens ein Überdruck auf das Innere des Rohrs aufgebracht wird.

14. Verfahren nach Anspruch 12 oder Anspruch 13, weiterhin durch den Schritt gekennzeichnet, das Rohr wenigstens nach dem Schritt des Strangpressens zu erhitzen.

## Revendications

1. Ballon pour un cathéter de dilatation, comprenant un élément polymère tubulaire (20) formé pour s'aplatir sous l'influence d'une pression interne réduite en une configuration définie par au moins trois ailes (68) qui s'étendent longitudinalement, caractérisé en ce que le corps tubulaire (20) présente un segment d'une épaisseur réduite entre des ailes adjacentes (68) pour procurer un pli de repliage (66), le corps tubulaire (20) formant au moins trois plis (66) qui s'étendent longitudinalement et des ailes alternées (68).

2. Ballon suivant la revendication 1, caractérisé en ce que les segments sont des lignes qui s'étendent longitudinalement au ballon (20).

3. Ballon suivant l'une des revendications 1 et 2, caractérisé en ce que le ballon (20) est non élastique.

4. Ballon suivant la revendication 3, caractérisé en ce que le ballon (20) est formé de téréphtalate de polyéthylène.

5. Ballon suivant la revendication 3, caractérisé en ce que le ballon (20) est formé de polyéthylène.

6. Ballon suivant la revendication 3, caractérisé en ce que le ballon (20) est formé de chlorure de polyvinyle.

7. Cathéter de dilatation comprenant une tige flexible allongée (18) présentant au moins un passage (22) s'étendant à travers elle depuis son extrémité proximale jusqu'à son extrémité distale, un ballon de dilatation (20) monté sur l'extrémité distale de la tige (18) et permettant une communication de fluide avec le passage, et un raccord pour relier le passage avec un dispositif de gonflement et de dégonflement, le cathéter étant caractérisé en ce que le ballon (20) est tel que revendiqué dans l'une quelconque des revendications 1 à 6.

8. Cathéter de dilatation suivant la revendication 7, caractérisé en ce que le ballon, lorsqu'il est dégonflé, forme lesdits au moins trois plis (66) autour de la tige (18).

9. Cathéter de dilatation suivant l'une des revendication 7 et 8, caractérisé en ce que les segments sont des lignes de pliage qui s'étendent longitudinalement au ballon (20).

10. Cathéter de dilatation suivant l'une quelconque des revendications 7 à 9, caractérisé en ce que le cathéter comprend un cathéter d'angioplastie.

11. Cathéter de dilatation suivant la revendication 10, caractérisé en ce que le cathéter comprend un cathéter d'angioplastie coronaire transluminale percutanée.

12. Procédé de formation d'un ballon plissé pour cathéter de dilatation, caractérisé par les étapes d'extrusion d'un tube ayant au moins trois segments, qui s'étendent longitudinalement et qui une moindre épaisseur que le reste du tube pour former des plis (62, 64, 66) dedans, et d'étirage biaxial du tube (20) en une forme prédéfinie.

13. Procédé suivant la revendication 12, caractérisé en ce que l'étape d'étirage biaxial du tube comprend un étirage axial du tube pendant une application de pression positive à l'intérieur du tube au moins pendant l'étirage axial.

14. Procédé suivant l'une des revendications 12 et 13, caractérisé en outre par l'étape de chauffage du tube au moins après l'étape d'extrusion.
